# EUROPÄISCHE PATENTSCHRIFT

(11) **EP 0 658 585 B1**
(45) Veröffentlichungstag und Bekanntmachung des Hinweises auf die Patenterteilung: **19.07.2000**
(21) Anmeldenummer: 94118551.4
(22) Anmeldetag: 25.11.1994
(51) Int. Cl.: C08G 65/32, A61K 47/48

(54) **Amidinophenylalaninderivate, Verfahren zu deren Herstellung, deren Verwendung und diese enthaltende Mittel als Antikoagulantien**
Aminophenylalanine derivatives, process for their production, their use and agents containing them as anti-coagulants
Dérivés d'aminophénylalanine, procédé pour leur, production leur usage et agents contenant ceux-ci comme anticoagulants

(30) Priorität: 10.12.1993 DE 4342154
(43) Veröffentlichungstag der Anmeldung: 21.06.1995
(73) Patentinhaber: Stüber, Werner, 35094 Lahntal (DE)
(72) Erfinder: Stüber, Werner, Dr., D-35094 Lahntal 3 (DE); Koschinsky, Rainer, DI, D-35091 Cölbe (DE)

(56) Entgegenhaltungen:
- EP-A- 0 154 432
- EP-A- 0 345 616
- EP-A- 0 508 220
- EP-A- 0 513 543
- EP-A- 0 558 961
- FR-A- 2 593 813
- GB-A- 2 247 239

## Beschreibung

Die Erfindung betrifft Amidinophenylalaninderivate, die Synthese dieser Verbindungen, deren Verwendung sowie pharmazeutische Mittel, die diese, als Thrombininhibitoren wirksamen Verbindungen enthalten.

Wie in EP-A- 0 508 220 und 0 513 543 gezeigt ist, können Amidinophenylalaninderivate dazu dienen, durch Inhibition von Thrombin den pathologischen Vorgang einer Thrombose günstig zu beeinflußen. Für eine medizinische Anwendung derartiger Verbindungen ist es jedoch oft wünschenswert, die Verweildauer im menschlichen Körper zu verlängern.

Dieser Erfindung lag deshalb die Aufgabe zu Grunde, neue Derivate von Amidinophenylalanin zu finden, die eine verlängerte Verweilzeit im Organismus von Warmblütern aufweisen.

Überraschenderweise konnte die Aufgabe dadurch gelöst werden, daß Derivate des Amidinophenylalanins an polymere Substanzen aus der Gruppe der Polyethylenglykole gebunden wurden.

Geeignete Derivate des Amidinophenylalanins sind beispielsweise solche, wie sie in EP-A- 0 508 220 und 0 513 543 beschrieben sind.

Als Polymere aus der Gruppe der Polyethylenglykole kommen physiologisch verträgliche Substanzen in Frage, die kovalent an die inhibitorisch aktiven Substanzen gebunden sind. Solche Polymere Polyethylenglykole (PEG) haben eine Molmasse von bis zu 75000, vorzugsweise von etwa 750-30000, vorzugsweise 750-20 000.

Demnach betrifft die Erfindung Substanzen der Formel I 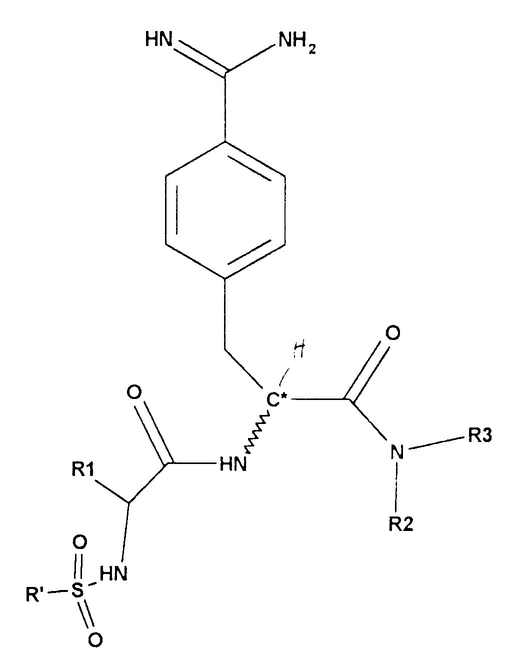 worin
- R': ein in der alpha- oder beta-Position gebundener Naphthalinring ist, der gegebenenfalls mit Alkylgruppen, die bis zu 3 C-Atomen enthalten, und/oder
Alkoxygruppen mit jeweils bis zu 3 C-Atomen, derivatisiert ist,
oder ein in der alpha- oder beta-Position gebundener Tetralinring ist, der gegebenenfalls mit Alkylgruppen, die bis zu 3 C-Atomen enthalten, und/oder Alkoxygruppen mit jeweils bis zu 3 C-Atomen, derivatisiert ist,
oder ein Phenylring ist, der gegebenenfalls mit Alkylgruppen, die bis zu 4 C-Atomen enthalten, und/oder mit bis zu drei Gruppen der Struktur O-X, in der O Sauerstoff oder Schwefel und X Wasserstoff, Methyl, Ethyl, n-Propyl, i-Propyl oder tert.-Butyl derivatisiert ist oder ein Chromansystem ist, das vorzugsweise mit bis zu 5 Alkylgruppen, die bis zu 3 C-Atomen enthalten, derivatisiert ist,
- R 1: eine Gruppe der Struktur A-B-Poly ist, worin A -(CH₂)ₙ- bedeutet, n eine ganze Zahl von 1 bis 7 ist, B eine Bindung,
-CO-NH-, -N(Y)-CO- mit Y = H, CH₃ oderC₂H₅, -CO-O-,-O-CO-, -SO₂-NH-, -S-S-, -S-, -O- oder -N(Y)- mit Y = H, CH₃ oderC₂H₅ bedeudet und
Poly ein Polymer aus der Gruppe der Polyethylenglykole ist, und dieses Polymer eine Molmasse von bis zu 75 000 hat, und
- R2 und R3: gleich oder verschieden sein können und eine Alkylgruppe mit bis zu 4 C-Atomen sind oder zusammen einen Ring bilden und eventuell mit einer Hydroxylgruppe oder einer Hydroxyalkylgruppe, wobei die Hydroxyalkylgruppe bis zu 3 C-Atome enthält derivatisiert sein können und diese Hydroxylgruppe gegebenenfalls auch verestert vorliegt, wobei die entsprechenden Säuren Carbonsäuren sind, die vorzugsweise bis zu 17 C-Atomen enthalten,
sowie deren physiologisch vertretbaren Salze und
- C *: in der R oder S Struktur, vorzugsweise aber in der R- Struktur vorliegt.

Im näheren Sinne und ohne Beschränkung auf die nachfolgenden Beispiele, eignen sich als R' bevorzugt die folgenden Gruppen:
- 6,7-Dimethoxynaphthyl-: (β-Dmn)
- 5-Methoxynaphthyl-: (β-Mns)
- 2,2,5,7,8-Pentamethylchromanyl-: (Pmc)
- 5,6,7,8-Tetrahydronaphthyl-: (Thn)
- 5,6,7,8-Tetramethylnaphthyl-: (Tmn)
- Phenyl- (Phl):
- 4-Methoxy-2,3,6-trimethylphenyl-: (Mtr)
- 2,3,4,5,6-Pentamethylphenyl-: (Pme)
- 4-Methoxy-2,3,5,6-tetramethylphenyl-: (Mte)und
- 4-Hydroxy-2,3,6-trimethylphenyl-: (Htr)
n ist vorzugsweise 1,2 oder 3.

Als Polymere werden Polyethylenglykole (PEG) benutzt.

B = -CO-NH- ist bevorzugt. Das bedeutet, an die Gruppe R1 mit -CH₂-COOH ist ein Polymer mit einer Aminofunktion angekoppelt. Solche Kopplungsreaktionen sind literaturbekannt. Beispielweise dient dazu eine Kopplungsreaktion mit einem Carbodiimid. Aminogruppen enthaltende Polymere, beispielsweise Amino-PEG der Struktur NH₂-CH₂-CH₂-PEG-OCH₃ (MM 750-20000) sind kommerziell erhältlich (RAPP POLYMERE, TÜBINGEN). Andererseits ist es auch möglich, ein Polymer über eine Carboxylgruppe an eine Aminogruppe des Restmoleküls (Gruppe B = - NH-CO-) zu binden. Dazu wird beispielsweise ein Polymer der Struktur CH₃O-PEG-CH₂-CH₂-NH-CO-CH₂-CH₂-COOH an die Seitenkette R1 = -(CH₂)ₙ-N(Y)H, wobei n und Y die oben genannte Bedeutung haben, gebunden. Desweiteren können Polymere der Struktur CH₃O-PEG-CH₂-CH₂-Br oder CH₃O-PEG-CH₂-CH₂-SH mit Verbindungen der Formel I,
worin R1 W-(CH₂)ₙ-
mit W = SH, OH oder NH(Y) und n wie zu Formel I angegeben ist, zu erfindungsgemäßen Verbindungen mit B ist S, O oder N(Y) mit Y = H, CH₃ oder C₂H₅ oder S-S umgesetzt werden.

Sind an den Resten R2 oder R3 Hydroxylfunktionen vorhanden, so können diese gegebenenfalls mit Carbonsäuren verestert sein. Solche Carbonsäuren sind beispielsweise Essigsäure, Bernsteinsäure, Hexancarbonsäure, Oktancarbonsäure, Dekancarbonsäure, Dodekancarbonsäure oder Hexadekancarbonsäure.

Da die Amidinofunktion auf Grund der basischen Wirkung in der Regel als Salz vorliegt, können die erfindungsgemäßen Thrombininhibitoren auch in unterschiedlichen Salzformen vorkommen. Die Salzform hat dabei einen wesentlichen Einfluß auf die Löslichkeit der Verbindungen sowie auf die Resorbierbarkeit im Falle der therapeutischen Anwendung. Als Salzformen seien genannt Formiate, Acetate, Caproate oder Oleate, das heißt Carbonsäuren mit bis zu 16 C-Atomen, Chloride, Bromide, Jodide, Alkansulfonate mit bis zu 10 C-Atomen, Salze von Dicarbonsäuren oder Tricarbonsäuren wie Citrate oder Tartrate.

Zum Gegenstand der Erfindung zählt auch die Herstellung der polymergebundenen Verbindungen. Bevorzugt wird ein Aminosäurederivat der Formel II 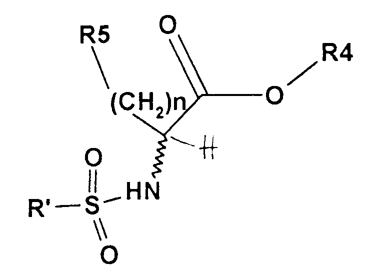 wobei R' und n obige Bedeutung haben und R₄ eine in der Aminosäurechemie übliche Schutzgruppe ist, beispielsweise eine Benzylgruppe, eine tert-Butylgruppe oder eine Methylgruppe und R₅ eine Carboxylgruppe oder eine Aminogruppe, eine Hydroxylgruppe, eine SO₃H-Gruppe oder eine Sulfhydrylgruppe (-SH) darstellt, mit einem oben genannten Polymer aus der Gruppe der Polyethylenglykole umgesetzt. Die Herstellung erfolgt nach an sich bekannten Reaktionswegen und führt nach Abspaltung der Schutzgruppe R4 zu einem polymergebundenen Aminosäure-Derivat der Formel II,
worin R5 eine Gruppe Polymer-B- darstellt, R4 Wasserstoff bedeutet und

R', n und B die oben genannte Bedeutung haben. Diese polymergebundene Aminosäure wird nach bekannten Kopplungsmethoden, beispielsweise mit Dicyclohexylcarbodiimid im Lösemittel Dimethylformamid, an die primäre Aminogruppe einer Verbindung der Formel III 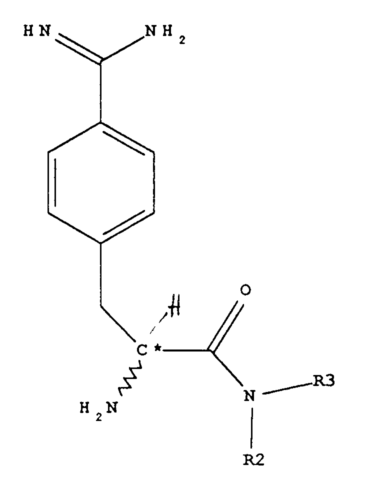 gebunden und somit ein erfindungsgemäßer Stoff erhalten.

Die erfindungsgemäßen Substanzen sind wertvolle Thrombininhibitoren, die sich besonders dadurch auszeichnen, daß sie eine verlängerte Halbwertszeit in Plasma besitzen. Diese Substanzen sind auch deshalb sehr wertvoll, weil sie beispielsweise bei subkutaner Gabe einen langsamen Anstieg ihrer Konzentration im Plasma zeigen. Zusammen mit einer längeren Halbwertszeit wird insgesamt eine deutlich verlängerte antithrombotische Wirkung erreicht.

Entsprechend der Lehre in PEPTIDE RESEARCH Vol.6 No. 3, 140-146 (1993), würde der Fachmann von solchen polymergestützten Verbindungen erwarten, daß die Bindefähigkeit gegenüber dem Thrombin, ausgedrückt durch den Ki-Wert herabgesetzt wird. Überraschenderweise wird die Potenz der Inhibitoren durch Anbindung der polymeren Träger aber signifikant erhöht.

Ebenfalls überraschend ist, daß die Verträglichkeit der erfindungsgemäßen Verbindungen durch die Polymeranbindung verbessert wird.

Die erfindungsgemäßen Inhibitoren wurden zur Beurteilung ihrer Wirksamkeit nach verschiedenen Kriterien geprüft,
vorzugsweise waren dies die Bestimmung des Ki-Wertes,
des IC₅₀-Wertes und der partiellen Thromboplastinzeit (PTT) in vivo und in vitro. Zur Spezifitätsprüfung wurden die IC₅₀-Werte und Ki-Werte gegen diverse Serinproteasen, insbesondere Thrombin und Trypsin, ermittelt. Die Stabilität der erfindungsgemäßen Substanzen wurde dadurch bestimmt, indem eine Substanzprobe mit einem Reinenzym, bevorzugt Trypsin, Chymotrypsin oder Papain, sowie mit Leber- oder Darmhomogenaten inkubiert wurde, aus den Lösungen in zeitlichen Abständen Proben entnommen wurden und vorzugsweise mit HPLC gemessen wurde.

Bei den beanspruchten Verbindungen handelt es sich um spezifische und hochaktive Thrombininhibitoren mit einem beträchtlichen antithrombotischen Potential, das die bisher bekannten niedermolekularen Inhibitoren hinsichtlich Wirkdauer und Wirksamkeit übertrifft.

Abkürzungen:
- Aph: Amidinophenylalanin
- NAPAP: β-Naphthylsulfonyl-glycyl-D,L-p-amidinophenylalanyl-piperidid
- Asp: Asparaginsäure
- Asn: Asparagin
- Glu: Glutaminsäure
- Cys(SO₃H): Cysteinsulfonsäure
- β-Dmn: 6,7-Dimethoxynaphthyl-
- β-Mns: 5-Methoxynaphthyl-
- Pmc: 2,2,5,7,8-Pentamethylchromanyl-
- Thn: 5,6,7,8-Tetrahydronaphthyl-
- Tmn: 5,6,7,8-Tetramethylnaphthyl-
- Phl: Phenyl-
- Mtr: 4-Methoxy-2,3,6-trimethylphenyl-
- Pme: 2,3,4,5,6-Pentamethylphenyl-
- Mte: 4-Methoxy-2,3,5,6-tetramethylphenyl-
- Htr: 4-Hydroxy-2,3,6-trimethylphenyl-
- Cph: 4-Carboxyphenyl-
- Z (Cbo): Benzyloxycarbonyl-
- Boc: tert.-Butyloxycarbonyl
- Fmoc: Fluorenylmethyloxycarbonyl
- Pip: Piperidin
- OtBu: tert.-Butylester
- OMe: Methylester
- OEt: Ethylester
- OiBu: iso-Butylester(sec-Butylester)
- OiPr: iso-Propylester
- DC: Dünnschichtchromatographie
- DCCI: Dicyclohexylcarbodiimid
- DMF: Dimethylformamid
- FAB-MS: Fast atom bombardment mass spectrometry
- TOTU: O-[(Cyano(ethoxycarbonyl)methyliden) amino]-1,1,3,3-tetramethyluronium-tetrafluoroborat
- DIPEA: N-Ethyldiisopropylamin

Die folgenden Beispiele beschreiben die Erfindung näher:

### Beispiel 1:

### 4-Methoxy-2,3,6-trimethylphenylsulfonyl-L-Asp(PEG5000)-D-p-amidinophenylalanyl -piperidid

### 1)4-Methoxy-2,3,6-trimethylphenylsulfonyl-L-Asp(PEG5000)-OH

***A)***
   4g Amino-PEG 5000-monomethylether wurden mit 400 mg (1mmol) 4-Methoxy-2,3,6-trimethylphenylsulfonyl-L-Asp-α-tert-butylester in 200ml DMF gelöst.
   Dazu wurden unter Rühren und Kühlung auf 0°C 328 mg (1mmol) TOTU als Festsubstanz und anschließend 0.2 ml DIPEA gegeben. Der Ansatz wurde 1h bei 0°C und 15h bei Raumtemperatur gerührt. Nach Abrotieren des Lösungs-mittels wurde der verbleibende Rückstand mehrmals mit Essigsäureethylester ausgerührt, in warmem Methanol gelöst und mit Diethylether auskristallisiert. Nach Absaugen und Trocknen wurde die Substanz in dieser Form für die nächste Reaktion eingesetzt.
***B)***
   Der unter ***A)*** hergestellte 4-Methoxy-2,3,6-trimethyl-phenylsulfonyl-L-Asp(PEG5000)-α-tert-butylester wurde in 100ml einer Lösung von 2 N HCL in Essigsäure gerührt, wobei eine klare Lösung entsteht. Anschließend wurde das Lösungsmittel im Vakuum abgezogen und anhaftende Säurespuren mittels Toluol im Vakuum abgedampft. Es wurde aus Methanol/Diethylether umgefällt und nach Trocknung ein weißer Feststoff erhalten.
   Ausbeute:2.8g
   Reinheitskontrolle: DC
      Rf=0.7(Butanol/Eisessig/Pyridin/Wasser 4/1/1/2)
   Drehwert:[α]²⁰D -1.8 (c=1, Methanol)
   Fp: 57°C

### 2)4-Methoxy-2,3,6-trimethylphenylsulfonyl-L-Asp(PEG5000)-D-4-aminidinophenylalanyl-piperidid

1.5g 4-Methoxy-2,3,6-trimethylphenylsulfonyl-L-Asp(PEG5000)-OH wurden mit 100 mg D-4-Amidinophenyl-alanylpiperidid in 50 ml DMF gelöst. Zu der auf 0°C abgekühlten Lösung wurde unter Rühren 100 mg TOTU gefolgt von 0.11 ml DIPEA gegeben. Nach einer Stunde bei 0°C wurde der Ansatz eine Stunde bei Raumtemperatur gehalten und anschließend das Lösungsmittel abrotiert. Der Rückstand wurde in Wasser gelöst und 3 mal mit Essigsäureethylester ausgeschüttelt, die wässrige Phase lyophilisiert und das Lyophilisat in Methanol an Sephadex^{R} LH20 chromatographiert.
Ausbeute:1.2g
Reinheitskontrolle: DC
   Rf=0.5(Chloroform/Methanol/Eisessig 50/10/2.5)
¹³C-NMR: (CD3OD) : 12.3, 18.4, 25.3, 26.6, 27.4, 30.5, 39.2, 40.4, 44.4, 51.1, 54.5, 56.3, 57.8, 71.6(groß), 113.47, 126.3, 128.0, 129.1, 130.5, 131.8, 140.2, 145.1, 160.9, 167.9, 169.6, 171.71

### Bestimmung der Inhibitionskonstanten für Thrombin

Die Inhibitionskonstanten (Ki) für die Substanzen wurden enzymkinetisch nach bekannten Verfahren bestimmt. Das eingesetzte humane Thrombin wurde als 87% rein mit Hilfe der "active site titration" bestimmt. Die Testlösung für die Ki-Bestimmung bestand aus Puffer (50 mM Tris-HCl, 75 mM NaCl, pH7.8, 37 Grad C), 100 pM Thrombin, 0.1 nM Substrat S2238 (Kabi) und Inhibitor, der einen Bereich von 0 bis 0.2 nM umspannte. Inhibitor und Enzym wurden 60 Minuten vorinkubiert, die Reaktion wurde gestartet durch Zugabe des chromogenen Substrats S2238. Für die Auswertung der Kinetiken wurde der mathematische Algorithmus für "tight-binding" verwendet, der mit Hilfe der nicht-linearen Regression Ki-Werte (Tabelle) und den Hemmtyp ergab. Der Hemmtyp für alle Inhibitoren wurde als kompetitiv ermittelt.

**TABELLE**

| **SUBSTANZ** | Kᵢ [nM]Thrombin | Kᵢ [nM]Trypsin | t_{1/2} β [min] |
|---|---|---|---|
| Mtr-Asp(PEG5000)-Aph-Pip (**1**) | 0.3 | 387 | * |
| Mtr-Asp(PEG5000)-Aph-Pip (**2**) | 0.4 | 585 | 33 |
| Mtr-Asp(PEG10000)-Aph-Pip(**3**) | 0.6 | 1500 | 63 |
| Mtr-Asp(PEG20000)-Aph-Pip(**4**) | 0.6 | 941 | 74 |
| Mtr-Asp-Aph-Pip (**5**) | 2.5 | 312 | 19 |

| | | | |
|---|---|---|---|
| * nicht getestet | | | |

### Bestimmung der Spezifität der Inhibitoren

Die Spezifität der Inhibitoren wurde gegenüber Thrombin und Trypsin bestimmt. Die Spezifität ist definiert als das Verhältnis der Ki-Werte für Trypsin und Thrombin (Tabelle).

### Bestimmung der Eliminations-Halbwertszeiten t_{1/2} β

Die Thrombininhibitoren (**2**)-(**5**) in der Tabelle wurden
in einer Dosierung von 5 mg/kg in Ratten (5 je Gruppe) intravenös appliziert. Es wurden Blutproben entnommen und die zeitabhängigen Konzentrationen an Thrombininhibitoren im Plasma bestimmt. Die Elimination erfolgt zweiphasig. Die berechneten Eliminations-Halbwertszeiten t_{1/2} β sind in der *Tabelle* zusammengestellt.

### Bestimmung der subkutanen Aufnahme der Substanzen (2) und (5)

Die Substanzen wurden in einer Dosierung von 2 mg/kg subkutan an Kaninchen verabreicht. Die maximale Plasmakonzentration von (**5**) wurde nach 5 Minuten erreicht. Die maximale Plasmakonzentration von (**2**) wurde nach 80 Minuten erreicht. (**2**) zeigte einen wirksamen Plasmaspiegel über 6 Stunden (**5**) über 3 Stunden.

## Patentansprüche

1. Verbindung der Formel I 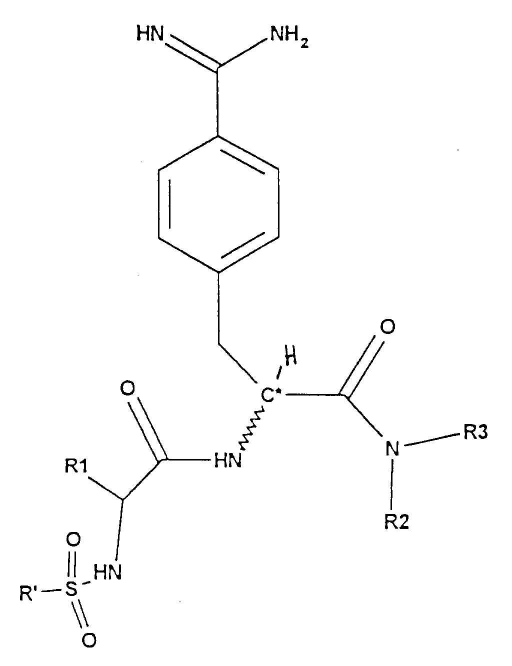 worin
R' ein in der alpha- oder beta-Position gebundener Naphthalinring ist, der gegebenenfalls mit Alkylgruppen, die bis zu 3 C-Atome enthalten, und/oder
Alkoxygruppen mit jeweils bis zu 3 C-Atomen, derivatisiert ist,
oder ein in der alpha- oder beta-Position gebundener Tetralinring ist, der gegebenenfalls mit Alkylgruppen, die bis zu 3 C-Atome enthalten₁ und/oder Alkoxygruppen mit jeweils bis zu 3 C-Atomen, derivatisiert ist,
oder ein Phenylring ist, der gegebenenfalls mit Alkylgruppen, die bis zu 4 C-Atome enthalten, und/oder mit bis zu drei Gruppen der Struktur O-X, in der O Sauerstoff oder Schwefel und X Wasserstoff, Methyl, Ethyl, n-Propyl, i-Propyl oder tert.-Butyl derivatisiert ist oder ein Chromansystem ist, das vorzugsweise mit bis zu 5 Alkylgruppen, die bis zu 3 C-Atome enthalten, derivatisiert ist,
R 1 eine Gruppe der Struktur A-B-Poly ist, worin
A -(CH2)n- bedeutet, n eine ganze Zahl von 1 bis 7 ist
B eine Bindung,
-CO-NH-, -N(Y)-CO- mit Y= H, CH₃ oder C₂H₅, -CO-O-, -O-CO-, -SO₂-NH-, -S-S-, -S-, -O- oder -N(Y)- mit Y = H, CH₃ oder C₂H₅ ist und
Poly ein Polymer aus der Gruppe der Polyethylenglykole bedeutet ist und dieses Polymer eine Molmasse von bis zu 75000 hat, und
R2 und R3 gleich oder verschieden sein können und eine Alkylgruppe mit bis zu 4 C-Atomen sind oder zusammen einen Ring bilden und eventuell mit einer Hydroxylgruppe oder einer Hydroxyalkylgruppe, wobei die Hydroxyalkylgruppe bis zu 3 C-Atome enthält derivatisiert sein können und diese Hydroxylgruppe gegebenenfalls auch verestert vorliegt, wobei die entsprechenden Säuren Carbonsäuren sind, die vorzugsweise bis zu 17 C-Atome enthalten,
sowie deren physiologisch vertretbaren Salze und
C * in der R oder S Struktur, vorzugsweise aber in der R-Struktur vorliegt.

2. Verbindung nach Anspruch **1**, dadurch gekennzeichnet, daß C * in der R Struktur vorliegt.

3. Eine Verbindung nach Anspruch **1**, in der R' β-Naphthyl, A = -CH₂- B = -CO-NH-, und Poly ein Polyethylenglykol mit einer Molmasse von 750 bis 20000 ist und R2 und R3 zusammen Piperidin sind.

4. Eine Verbindung nach Anspruch **1**, in der R' β-6,7-Dimethoxynaphthyl-, A = -CH₂-, B = -CO-NH-, und Poly ein Polyethylenglykol mit einer Molmasse von 750 bis 20000 ist und R2 und R3 zusammen Piperidin sind.

5. Eine Verbindung nach Anspruch **1**, in der R' β-Tetralinyl ist.

6. Eine Verbindung nach Anspruch **1**, in der R' 4-Methoxy-2,3,6-trimethylphenyl- ist.

7. Eine Verbindung nach Anspruch **1**, in der R' 2,2,5,7,8-Pentamethylchromanyl- ist.

8. Eine Verbindung nach Anspruch **1**, dadurch gekennzeichnet, daß B -N(Y)-CO- mit Y H, CH₃, oder C₂H₅ ist.

9. Verfahren zur Herstellung einer Verbindung der Formel I, dadurch gekennzeichnet, daß eine Verbindung der Formel II: 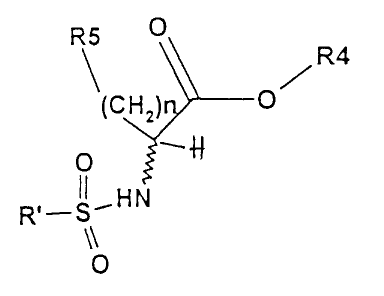 wobei R' und n obige Bedeutung haben und R4 eine in der Aminosäurechemie übliche Schutzgruppe ist, beispielsweise eine Benzylgruppe, eine tert-Butylgruppe oder eine Methylgruppe und R₅ eine Carboxylgruppe oder eine Aminogruppe, eine Hydroxylgruppe, eine SO₃H-Gruppe oder eine Sulfhydrylgruppe (-SH) darstellt, mit einem oben genannten Polymer aus der Gruppe der Polyethylenglykole umgesetzt wird, die Schutzgruppe R4 abgespalten wird, wobei ein polymergebundenes Aminosäure-Derivat der Formel II,
worin R5 eine Gruppe Polymer-B- darstellt, R4 Wasserstoff bedeutet und R', n und B die oben genannte Bedeutung haben, entsteht und dieses Derivat nach bekannten Kopplungsmethoden, an die primäre Aminogruppe einer Verbindung der Formel III 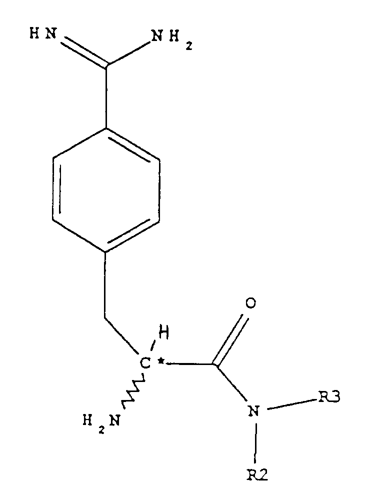 gebunden und eine Verbindung der Formel I in Anspruch **1** erhalten wird.

10. Diagnostisches oder therapeutisches Mittel enthaltend eine Verbindung nach Anspruch **1**.

11. Verwendung einer Verbindung nach Anspruch **1** zur Herstellung eines Diagnostikums oder eines Arzneimittels mit antithrombotischer Wirkung.

## Claims

1. A compound of formula I 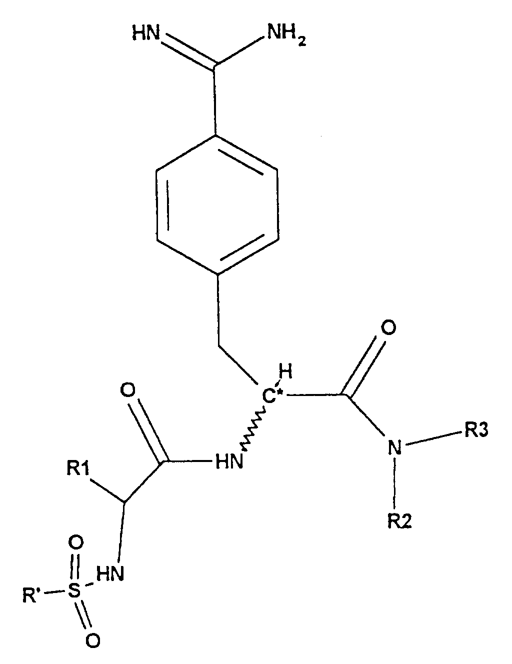 in which
R' is a naphthalene ring which is bonded in the alpha or beta position and is optionally derivatized with alkyl groups containing up to 3 carbon atoms and/or alkoxy groups having up to 3 carbon atoms in each case, or is a tetralin ring which is bonded in the alpha or beta position and is optionally derivatized with alkyl groups containing up to 3 carbon atoms and/or alkoxy groups having up to 3 carbon atoms in each case, or is a phenyl ring which is optionally derivatized with alkyl groups containing up to 4 carbon atoms and/or with up to three groups of the structure O-X in which O is oxygen or sulfur and X is hydrogen, methyl, ethyl, n-propyl, i-propyl, or tert-butyl, is a chroman system which is derivatized preferably with up to 5 alkyl groups which contain up to 3 carbon atoms
R 1 is a group of the structure A-B-Poly, in which A is -(CH2)n-, n is an integer from 1 to 7,
B is a bond, -CO-NH-, -N(Y)-CO- with Y= H, CH₃ or C₂H₅; -CO-O-, -O-CO-, -SO₂-NH-, -S-S-, -S-, -O- or -N(Y)- with Y = H, CH₃ or C₂H₅ and
Poly is a polymer from the group of polyethylene glycols and this polymer has a molecular weight of up to 75000, and
R2 and R3 can be identical or different and are an alkyl group with up to 4 carbon atoms or together form a ring and may possibly be derivatized with a hydroxyl group or a hydroxyalkyl group, where the hydroxyalkyl groups contain up to 3 carbon atoms, and this hydroxyl group is optionally also in esterified form, where the appropriate acids are carboxylic acids which preferably contain up to 17 carbon atoms, and their physiologically acceptable salts and
C * is in the R or S structure, but preferably in the R structure.

2. A compound as claimed in claim **1**, where in C * is in the R structure.

3. A compound as claimed in claim **1**, in which R' is β-naphthyl, A = -CH₂-, B = -CO-NH-, and poly is a polyethylene glycol with a molecular weight of 750 to 20000, and R2 and R3 together are piperidine.

4. A compound as claimed in claim **1**, in which R' is β-6,7-dimethoxynaphthyl-, A = -CH₂-, B = - CO-NH-, and poly is a polyethylene glycol with a molecular weight of 750 to 20000, and R2 and R3 together are piperidine.

5. A compound as claimed in claim **1**, in which R' is β-tetralinyl.

6. A compound as claimed in claim **1**, in which R' is 4-methoxy-2,3,6-trimethylphenyl.

7. A compound as claimed in claim **1**, in which R' is 2,2,5,7,8-pentamethylchromanyl.

8. A compound as claimed in claim **1**, in which B is -N(Y)-CO- with Y being H, CH₃ or C₂H₅.

9. A process for the preparation of a compound of formula I, which comprises reacting a compound of the formula II: 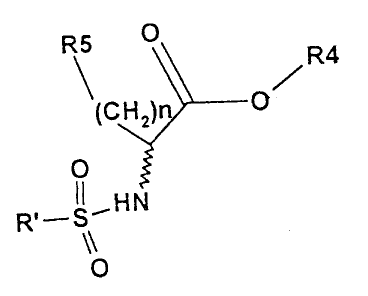 where R' and n have the above meaning, and R4 is a protective group customary in amino acid chemistry, for example a benzyl group, a tert-butyl group or a methyl group, and R₅ is a carboxyl group or an amino group, a hydroxyl group, a SO₃H group or a sulfhydryl group (-SH), with an above mentioned polymer from the group of the polyethylene glycols, eliminating the protective group R4, resulting in a polymer-bound amino acid derivative of the formula II, in which R5 is a group polymer-B-, R4 is hydrogen and R', n and B have the above mentioned meaning and bonding this derivative by known coupling methods to the primary amino group of a compound of the formula III 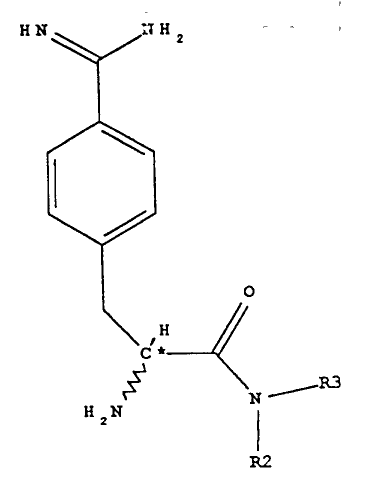 and obtaining a compound of the formula I in claim **1**.

10. A diagnostic or therapeutic composition containing a compound as claimed in claim **1**.

11. The use of a compound as claimed in claim **1** for the production of a diagnostic aid or of a pharmaceutical with antithrombotic effect.

## Revendications

1. Composé de formule I 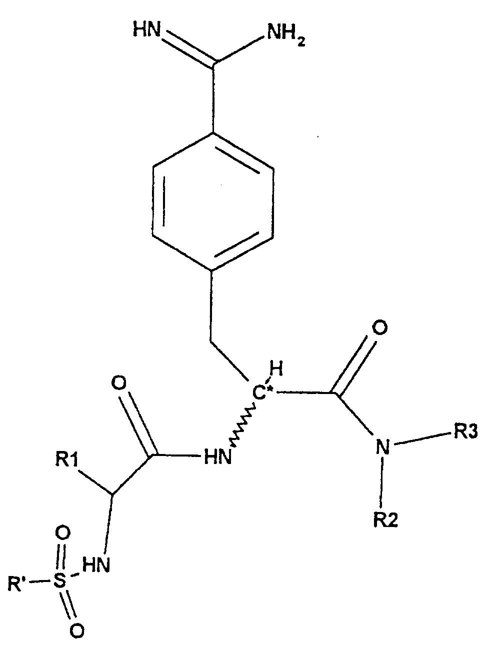 dans laquelle
R' représente un noyau naphtyle qui est lié en position alpha ou béta et est éventuellement substitué avec des groupements alkyle possédant jusqu'à trois atomes de carbone et/ou des groupements alkoxy possédant jusqu'à trois atomes de carbone chacun ou est un noyau tétraline qui est lié en position alpha ou beta et est éventuellement substitué avec des groupements alkyle possédant jusqu'à trois atomes de carbone et/ou des groupements alkoxy possédant respectivement jusqu'à trois atomes de carbone chacun, ou représente un noyau phenyle qui est éventuellement substitué avec des groupements alkyle possédant jusqu'à quatre atomes de carbone et/ou avec jusqu'à trois groupements de structure O-X dans laquelle O est un oxygène ou un soufre et X est un hydrogène, un groupement méthyle, éthyle, n-propyle, i-propyle, ou tert-butyle ou, est un système chromane qui est de préférence substitué avec jusqu'à cinq groupements alkyle qui contiennent jusqu'à trois atomes de carbone.
R1 représente un groupement de structure A-B-Poly, dans lequel A est -(CH2)n-, n est un entier de 1 à 7,
B est une liaison, -CO-NH-, -N(Y)-CO- avec Y=H, CH₃ ou C₂H₅, -CO-O-, -O-CO-, -SO₂-NH-, -S-S-, -S-, -O- ou -N(Y)- avec Y=H, CH₃ ou C₂H₅ et
Poly est un polymère issu du groupe des polyéthylène glycols et ce polymère un poids moléculaire jusqu'à 75 000, et
R2 et R3 peuvent être identiques ou différents et sont un groupe alkyle avec jusqu'à quatre atomes de carbone ou forment ensemble un cycle et peuvent éventuellement être substitués avec un groupe hydroxyle ou hydroxyalkyle, les groupements hydroxyalkyle contenant jusqu'à trois atomes de carbone, et ce groupe hydroxyle étant éventuellement également sous une forme estérifiée, les acides appropriés étant des acides carboxyliques qui de préférence contiennent jusqu'à 17 atomes de carbone, et leurs sels physiologiquement acceptables et
C* est de configuration R ou S, mais de préférence en configuration R.

2. Composé tel que revendiqué en revendication 1, dans laquelle C* est de configuration R.

3. Composé tel que revendiqué en revendication 1, dans laquelle R' est β-naphtyle, A = CH₂-, B = -CO-NH-, et poly est un polyéthylène glycol de poids moléculaire de 750 à 20 000, et R2 et R3 forment ensemble une pipéridine.

4. Composé tel que revendiqué en revendication 1, dans laquelle R' est un β-6,7-diméthoxynaphtyle-, A = CH₂-, B = -CO-NH-, et poly est un polyéthylène glycol de poids moléculaire entre 750 à 20 000, et R2 et R3 forment ensemble une pipéridine.

5. Composé tel que revendiqué en revendication 1, dans lequel R' est β-tétraline.

6. Composé tel que revendiqué en revendication 1, dans lequel R' est un 4-méthoxy-2,3,6-triméthylphényle.

7. Composé tel que revendiqué en revendication 1, dans lequel R' est un 2,2,5,7,8-pentaméthylchromanyle.

8. Composé tel que revendiqué en revendication 1, dans lequel B est -N(Y)-CO- avec Y étant H, CH₃ ou C₂H₅.

9. Procédé pour la préparation d'un composé de formule 1, qui comprend la réaction d'un composé de formule II. 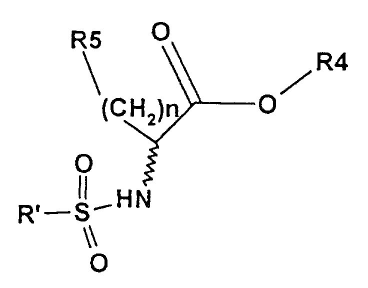
dans laquelle R' et n ont les significations précédentes, et R4 est un groupement protecteur usuel dans la chimie des acides aminés, par exemple un groupement benzyle, un groupement terbutyle ou un groupement méthyle et R5 est un groupement carboxyle ou un groupement amino, un groupement hydroxyle, un groupement SO₃H ou un groupement sulfhydryle (-SH), avec un polymère tel que défini ci-dessus choisi dans le groupe des polyéthylène glycols, l'élimination du groupement protecteur R4, de manière à conduire à un dérivé acide aminé lié à un polymère de formule II, dans laquelle R5 est un groupement polymère-B-, R4 est un hydrogène et R', n et B ont les définitions mentionnées ci-dessus, et, la liaison de ce dérivé à l'aide de méthodes de couplage connues, au groupement amino primaire d'un composé de formule III 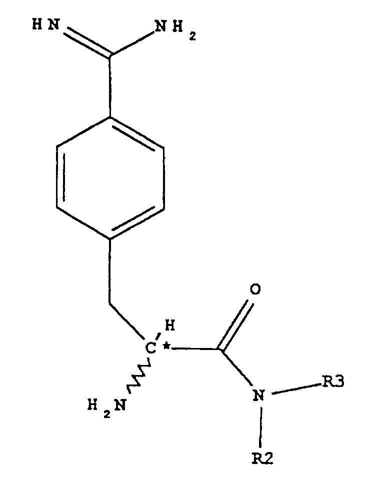 et l'obtention d'un composé de formule I tel que défini en revendication 1.

10. Composition diagnostique ou thérapeutique contenant un composé tel que défini en revendication 1.

11. Utilisation d'un composé tel que défini en revendication 1 pour la production d'un agent diagnostique et/ou d'un agent pharmaceutique à effet antithrombotique.
